(19) Europäisches Patentamt
European Patent Office
Office européen des brevets

(11) Publication number: **0 017 170**
**B1**

(12) **EUROPEAN PATENT SPECIFICATION**

(45) Date of publication of patent specification: **13.04.83**

(51) Int. Cl.³: **C 07 D 501/04,**
C 07 D 501/57

(21) Application number: **80101634.6**

(22) Date of filing: **27.03.80**

(54) **Improved process for the preparation of cephamycin antibiotics.**

(30) Priority: **30.03.79 US 25293**
**30.03.79 US 25294**

(43) Date of publication of application:
**15.10.80 Bulletin 80/21**

(45) Publication of the grant of the patent:
**13.04.83 Bulletin 83/15**

(84) Designated Contracting States:
**AT BE CH DE FR GB IT LU NL SE**

(56) References cited:
**US - A - 4 014 873**

**The file contains technical information submitted after the application was filed and not included in this specification**

(73) Proprietor: **MERCK & CO. INC.**
**126, East Lincoln Avenue P.O. Box 2000**
**Rahway New Jersey 07065 (US)**

(72) Inventor: **Love, George M.**
**1164 Wyoming Drive**
**Mountainside, New Jersey 07092 (US)**
Inventor: **Sohar, Paul**
**14 Sydenham Road**
**Warren, New Jersey 07060 (US)**
Inventor: **Weinstock, Leonard M.**
**P.O. Box 218**
**Belle Mead, New Jersey 08502 (US)**

(74) Representative: **Abitz, Walter, Dr.-Ing. et al,**
**Abitz, Morf, Gritschneder P.O. Box 86 01 09**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 017 170 B1

Improved process for the preparation of cephamycin antibiotics

RELATIONSHIP TO THE PRIOR ART:

The first disclosure in the patent literature of transacylation of Cephamycin C is in U.S. Patent No. 4,014,873, issued March 29, 1977. This process was conducted in the presence of a silylating agent. An improved transacylation, utilizing molecular sieves is claimed in U.S. Patent No. 4,053,286, issued October 11, 1977. The general chemistry of the transacylation reaction is in Weinstock et al., "The Chemistry of Cephamycins IV. Acylation of Amides in the Presence of Neutral Acid Scavengers", *Tet. Letters,* 46, 3979 (1975).

The patent, U.S. Patent 4,014,873, discloses silyl urethane reagents identical to those of the instant application, that is

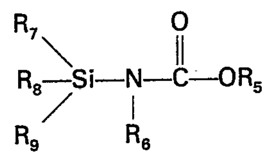

I

(Formula H, Col. 3) wherein $R_5$, $R_7$, $R_8$ and $R_9$ could be alkyl 1—8, and $R_6$ was hydrogen. A particular silyl urethane named included N-(trimethylsilyl)-ethyl carbamate, also called N-trimethylsilyl ethyl urethan, Col. 9, line 17, and N-trimethylsilyl methyl urethane, Col. 9, lines 17—18. The particular silyl enol ethers of this invention were not disclosed in this patent.

The process disclosed in that patent was primarily directed to an acylation, e.g., with thienylacetyl chloride of Cephamycin C, the latter being a fermentation product prepared from *Streptomyces lactamdurans* NRRL 3802, on permanent unrestricted deposit. Under certain conditions, (such as prolonging the reaction time, Col. 9, line 38), this acylation was taught to proceed in one step, see Col. 9, lines 35 to Col. 10, line 7. None of the examples actually used the silyl urethanes of the above formula. When the silylating agent was used in the reaction, a molecular excess was employed, see Col. 38, lines 44—45, in Claim 1.

SUMMARY OF THE INVENTION:

It has now been discovered that using about an equivalent amount of a silyl urethane or a trimethyl silyl enol ether in the acylation reaction, a significantly higher, unexpected yield of final product can be quickly and easily recovered.

THE SILYL URETHANE REAGENT:

The silyl urethane of this invention is a trimethyl silyl loweralkyl carbamate,

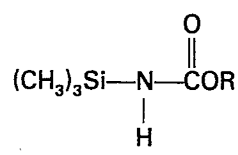

IA

where R is 1—4 carbon atoms, such as methyl, ethyl, or n-butyl. Particularly preferred is trimethylsilyl methyl carbamate.

With this reagent, about .8 to about 1.5 equivalents of the trimethylsilyl methyl carbamate, relative to Cephamycin C starting material is the optimal level to produce the final product in high yield.

THE TRIMETHYL SILYL ENOL ETHER REAGENT

The trimethylsilyl enol ether of this invention is:

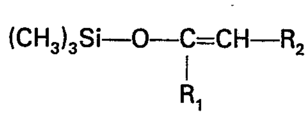

II

wherein $R_1$ can be lower alkyl of 1—4 carbon atoms; $R_2$ is hydrogen or lower alkyl of 1—4 carbon atoms; or $R_1$ and $R_2$ can be a joined alkylene chain of 2—4 carbon atoms. Particularly preferred compounds are those in which $R_2$ is H and $R_1$ is $CH_3$, or 2-trimethylsiloxyl propene; or $R_1$ and $R_2$ are a joined alkylene chain of 4 carbon atoms, or 1-trimethylsiloxyl cyclohexene.

These trimethylsilyl enol ethers of Formula II are made by reacting an appropriate ketone

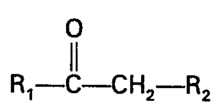

with trimethylsilyl chloride in the presence of base, such as sodium hydride. This chemistry is in the literature, see Hudrlik A. Takacs, *J. Org. Chem. 43,* 3861 (1978).

With this reagent, about .8 to 4 equivalents of the trimethylsilyl enol ether, relative to Cephamycin C starting material is the optimal level to produce the final product in high yield.

Both reagents share the same general conditions and the reaction is conducted at about 80—90°C, preferably 85°C, so that the internal gage pressure of reaction is about 2.1—3.2 kg/cm²., preferably about 2.8 kg/cm². The reaction progress is monitored by liquid chromatographic assay of the amount of imide, or Cephamycin C having an thienylacetyl side chain at the 7-amino group in addition to its normal adipoyl group. When the imide level is at 2—5% the reaction is essentially complete (the theoretical level of 0% is probably reached, but is difficult to measure accurately); the reaction is then cooled quickly to from about 85°C to about —10°C, in order to quench. The final product is then recovered. Generally, this reaction takes place in from 2—10 hours.

Although this reaction is best illustrated in the reacton of thienylacetyl chloride with Cephamycin C, a more generalized scheme is possible. Any acyl group of a 7-acylamido cephalosporin can be exchanged for another. The groups and definitions of breadth of this reaction are all those of U.S. Patent 4,014,873, the contents of which are incorporated by reference.

A preferred embodiment is illustrated in the following flow sheet.

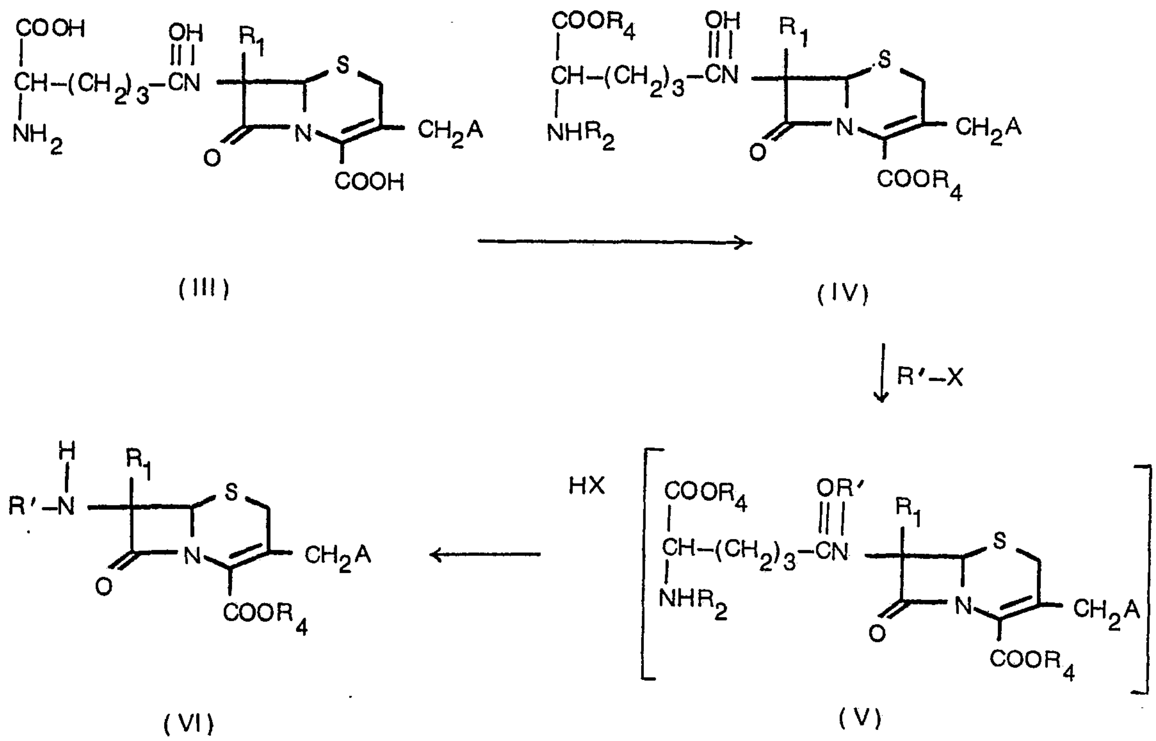

(III)
(IV)
(V)
(VI)

In the formulas of the above flowsheet, $R_1$ represents hydrogen or methoxy; A is as defined above, most desirably, acetoxy or carbamoyloxy; R' represents an acyl group as defined above; $R_4$ represents hydrogen or a blocking or protecting substituent; and $R_2$ represents hydrogen or an amino blocking or protecting substituent.

The side chain amino protecting group, $R_2$, in compound (V) does not have to be easily removable since the side chain is removed in the transacylation process. In fact, it is preferred that the side chain amino protecting group be one that is not easily removed since these are usually less expensive and more stable to handling during manufacturing.

In accordance with this process, the amino group of the starting cephalosporin compound (III) is first blocked ($R_2$) by reaction with a suitable reagent to protect the 5'-amino-substituent of the aminoadipoyl side chain. Thus, the amino group is blocked by amino protecting groups such as acyl, aroyl, alkoxycarbonyl, alkylsulfonyl, arylsufonyl, and the like in accordance with methods well known in this art. Specific groups suitable for blocking the amino group that might be mentioned are those wherein $R_2$ is trichloroethoxycarbonyl, tertiary butoxycarbonyl, benzoylmethoxycarbonyl, trimethylsilyl, p-methoxybenzyloxy, 2-nitrophenylsulfenyl, 2,4-dinitrophenylsulfenyl, chloroacetyl, p-nitrophenylthio, p-nitrobenzensulfonyl, p-toluenesulfonyl, methanesulfonyl, benzoyl, p-chlorobenzoyl, p-nitrobenzoyl, toluoyl, and the like, although we generally prefer to utilize the p-toluenesulfonyl or benzoyl derivative which is conveniently prepared by reacting the cephalosporin compound with p-toluenesulfonyl chloride or benzoyl chloride while keeping the pH of the mixture basic, i.e., between 9 and 10.

It is generally preferred to carry out the above-described reactions with a cephalosporin compound, (IV), wherein the carboxy groups on the aminoadipoyl side chain, and at the 4-position are likewise blocked or protected since maximum yields of the desired product are obtained with such derivatives. Although the carboxy group on the aminoadipoyl side chain is not necessarily deblocked, since it is removed in the cleavage step, the blocking or protecting group $R_4$ at the 4-position is preferably one which can be removed easily to obtain the free acid without disruption of the $\beta$-lactam group since the cephalosporin compounds are usually used in the form of salts such as alkali metal salts or an amine salt. Protecting groups suitable for this purpose are well known in this art. The methoxymethyl group is particularly preferred. In a preferred embodiment of this invention, the methoxymethyl group is cleaved by mixing the products with excess water.

The protected cephalosporin compound is then reacted with an acylating agent, R'—X, in a homogeneous solution in the presence of the trimethylsilyl methyl carbamate described above to obtain the diacylimide product (V). The acylating agent can be an acid halide (chloride or bromide), a functional equivalent thereof such as an acid anhydride, a mercaptide, a mixed acid anhydride with other carboxylic acids, an activated ester of the carboxylic acid such as the p-nitrophenyl ester, and the like. Thienylacetyl chloride is preferred.

The acylating agent is employed in amounts in molecular excess of that of the starting cephalosporin, preferably from 1 to 6 times as much acylating agent as cephalosporin, preferably in the range of 1 to 4 molar excess or most preferably in the range of 2 to 4 molar excess.

The silyl urethane is used in an amount approximately equivalent to the starting cephalosporin IV, or from .8 to 1.5 equivalents.

The trimethylsilyl enol ether is used in an amount from about 0.8 to 4 equivalents to the starting cephalosporin IV.

The acylation reaction takes place in a suitable solvent medium. The temperature at which this reaction is carried out is preferred to be from about 50°C. to 90°C. Various solvents which do not contain an active hydrogen such as chloroform, acetonitrile, methylene chloride, dioxane, benzene, halobenzene, carbon tetrachloride, 1,2-dichloroethane, and diethylether are most suitable as medium for the reaction mixture. The preferred solvent is methylene chloride. If desired, the reaction mixture is kept in motion by stirring or agitating during the reaction.

The cleavage to final product VI, under the conditions of solvent, temperature, amount of trimethylsilyl methyl carbamate, takes place spontaneously, due to liberation of acid from the reaction mixture products. The progress of reaction is monitored by decreased amounts of imide V, using standard liquid chromatographic or UV techniques. Generally, the reaction is completed within 2—10 hours, as evidenced by a drop in imide level to less than 5%.

This invention is illustrated by the following examples.

## Example 1

500 ml of a methylene chloride concentrate (dried to a Karl Fischer analysis of 0.08 g) of dimethoxymethyl ester of 7$\beta$ - (D - 5 - tosylamino - 5 - carboxylvaleramido) - 3 - carbamoyloxymethyl - 7 - methoxy - 3 - cephem - 4 - carboxylic acid (54.4 mmoles) 13 ml (12 g, 81.6 mmoles, if pure) of trimethylsilyl methyl carbamate, and 27.2 ml (34.9 g, 217 mmoles, 4 eq.) of thienylacetyl chloride were sequentially charged to a 1 l. stirred autoclave and heated to 85°C to afford an internal gage pressure of 2,8 kg/cm². Samples were withdrawn at hourly intervals and assayed by 1c.

lc mole %

| Hour | Sym Imide | Thienyl Product MM | Unsym. Imide | Theinyl Product Anhy |
|---|---|---|---|---|
| 1 | 11 | 48 | 18 | — |
| 2 | 14 | 61 | 8 | 1 |
| 3 | 9 | 73 | 4 | 2 |
| 4 | 3 | 76 | — | 3 |

| Hour | Starting Material MM | Mass Bal. | Total Imide |
|---|---|---|---|
| 1 | 31 | 108 | 29 |
| 2 | 16 | 100 | 22 |
| 3 | 12 | 100 | 11 |
| 4 | 9 | 91 | 3 |

The batch was quickly cooled at exactly 4.0 hours since this appeared to be the zero imide point. After cooling to 20°C, 447 g. (35.0 mmoles) of the batch was transferred from the autoclave to a 2 l. three neck flask with an overhead stirrer and cooled to —10°C. Seventy ml of ethanol was added and the mxiture was aged for 30 minutes followed by the addition of 700 ml of room temperature water. The pH of the two phase mixture was adjusted to 5.0 with 1 NNaOH solution, and the methylene chloride was removed under reduced pressure.

The two phase mixture was vigorously stirred overnight at 22°C while the pH was held at 5.0 by a pH meter switched pump which added 1 NNaOH solution as necessary to hold this pH. The crude mixture was then sequentially extracted 3 times at pH's of 4.4, 3.8 and 3.5 with 350 ml of methylene chloride. Lc assay of the 980 ml aqueous layer after the third extraction, column feed, was 12.1 g indicating a yield of 79%, as the carboxylic acid.

*Isolation*

After standing overnight at 6°C, the assay of the column feed had fallen from 12.1 g/l to 11.8 g/l. The column feed was then isolated using an IRA—68 chloride cycle resin column, followed by concentration using an XAD—2 resin column which was eluted with ethyl acetate. A breakthrough of 2.8% occurred on the IRA—68 column. Disregarding this breakthrough, the yield to ethyl acetate rich cut was 94%, and the overall yield to final product free acid hydrate was 85% with 2.5% in the mother liquors. Final product assays are given below.

| | |
|---|---|
| 1 c uncorrected purity, | 92.0% |
| EtOAc | 0.51% |
| HoAc | 0.53% |
| EtOH | 0.005% |
| KF | 4.30% |

Example 2

The advantage of using from 1 to 1.5 equivalents of the trimethylsilyl methyl carbamate is illustrated by comparing total imide levels at various times in hours for differing concentrations of the carbamate:

Mole % of Total Imide by 1c

| equiv. | 1 hour | 2 hours | 3 hours | 4 hours | 5 hours |
|---|---|---|---|---|---|
| 1.5 | 35 | 30 | 15 | 0 | — |
| 2.3 | 60 | 57 | 40 | 24 | 4 |
| 3.0 | 72 | 65 | 49 | 30 | 15 |

(These figures were obtained by graphing observed points on a chart and reading extrapolations.)

The high amounts of imide indicate the reaction proceeds more slowly, with consequent economic disadvantage. Since the peak total imide level is dependent on trimethylsilyl methyl carbamate charge, the level of 1 to 1.5 equivalents accommodates various problems with purity of starting material or traces of water while not affording overlong reaction times.

Example 3

500 ml of a methylene chloride concentrate (dried to a Karl Fischer analysis of 0.08 g) of dimethoxymethyl ester of 7β - (D - 5 - tosylamino - 5 - carboxylvaleramido) - 3 - carbamoyloxymethyl - 7 - methoxy - 3 - cephem - 4 - carboxylic acid (54.4 mmoles) 15.2 ml (13.2 g, 81.6 mmoles, if pure) of 2-trimethylsiloxyl cyclohexene, and 27.2 ml (34.9 g, 217 mmoles, 4 eq.) of thienylacetyl chloride were sequentially charged to a 1 l. stirred autoclave and heated to 85°C to afford an internal gage pressure of 2,8 kg/cm². Samples were withdrawn at hourly intervals and assayed by 1c.

1c mole %

| Hour | Sym Imide | Thienyl Product MM | Unsym. Imide | Thienyl Product Anhy |
|---|---|---|---|---|
| 1 | 13.1 | 42.2 | 30.9 | — |
| 2 | 16.7 | 61.3 | 12.4 | — |
| 3 | 9.3 | 72.3 | 5.5 | — |
| 4 | 0.5 | 79.4 | 0.2 | 2.4 |

| Hour | Starting Material MM | Mass Bal. | Total Imide |
|---|---|---|---|
| 1 | 24.5 | 111 | 44 |
| 2 | 16.7 | 107 | 29 |
| 3 | 12.9 | 100 | 15 |
| 4 | 11.3 | 94 | 0 |

The batch was quickly cooled at exactly 4.0 hours since this appeared to be the zero imide point. After cooling to 20°C, 429 g. (33.4 mmoles) of the batch was transferred from the autoclave to a 2 l. three neck flask with an overhead stirrer and cooled to −10°C. 67 ml of ethanol was added and the mixture was aged for 30 minutes followed by the addition 670 ml of room temperature water. The pH of the two phase mixture was adjusted to 5.0 with 1 NNaOH solution, and the methylene chloride was removed under reduced pressure.

The two phase mixture was vigorously stirred overnight at 22°C while the pH was held at 5.0 by a pH meter switched pump which added 1 NNaOH solution as necessary to hold this pH. The crude mixture was then sequentially extracted 3 times at pH's of 4.4, 3.8 and 3.5 with 350 ml of methylene chloride. Lc assay of the 958 ml aqueous layer after the third extraction, column feed, was 11.55 g/l indicating a yield of 77.5%, as the carboxylic acid.

*Isolation*

After standing overnight at 6°C, the assay of the column feed had fallen from 11.55 g/l to 11.17 g/l. The column feed was then isolated using an IRA—68 chloride cycle resin column, followed by concentration using an XAD—2 resin column which was eluted with ethyl acetate. A breakthrough of 2.9% occurred on the IRA—68 column. Disregarding this breakthrough, the yield to ethyl acetate rich cut was 91.5%, and the overall yield to final product free acid hydrate was 88% with 2.2% in the mother liquors. Final product assays are given below.

| | |
|---|---|
| 1 c uncorrected purity, | 93.0% |
| EtOAc | 0.4% |
| HoAc | 0.34% |
| EtOH | 0.003% |
| KF | 4.89% |

Example 4

Using a similar procedure to Example 1, except using 1-trimethylsiloxyl propene instead of 2-trimethylsiloxyl cyclohexane, a yield of 74.0% was achieved in the column feed.

**Claims**

1. In the process of preparing the compound

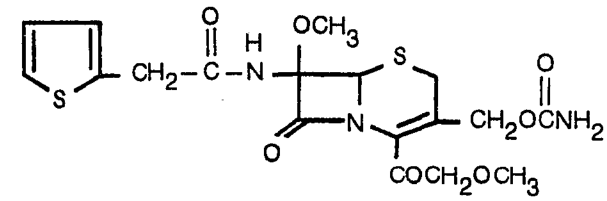

by reacting thienylacetyl chloride with

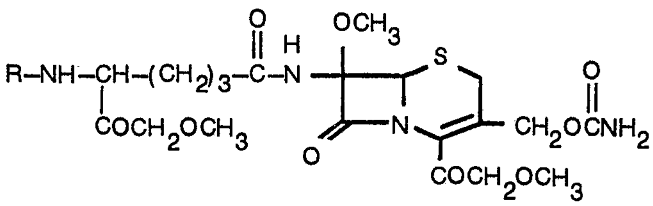

wherein R is p-tosyl in an inert solvent such as methylene chloride at a temperature of between 50—90°C, the improvement comprising carrying out the reaction in the presence of either (1) about .8 to 1.5 equivalents of trimethylsilyl loweralkyl carbamate or (2) about .8 to 4 equivalents of trimethylsilyl enol ether

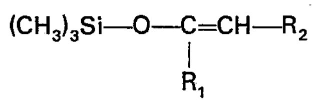 II

wherein $R_1$ can be lower alkyl of 1—4 carbon atoms; $R_2$ is hydrogen or loweralkyl of 1—4 carbon atoms; or $R_1$ and $R_2$ can be a joined alkylene chain of 2—4 carbon atoms;

2. The process of Claim 1 in which the trimethylsilyl loweralkyl carbamate has 1—4 carbon atoms in the loweralkyl.

3. The process of Claim 1 in which trimethylsilyl methyl carbamate is used.

4. The process of Claim 1 in which trimethylsilyl ethyl carbamate is used.

5. The process of Claim 1 in which the trimethylsilyl enol ether is 2-trimethylsiloxyl propene.

6. The process of Claim 1 in which the trimethylsilyl enol ether is 1-trimethylsiloxyl cyclohexene.

**Revendications**

1. Dans le procédé de préparation du composé

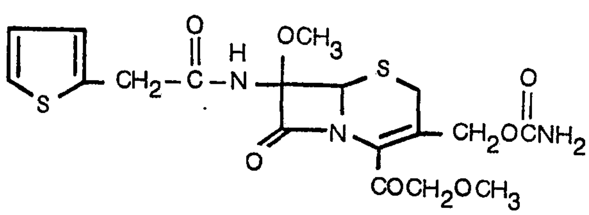

par réaction du chlorure de thiénylacétyle avec

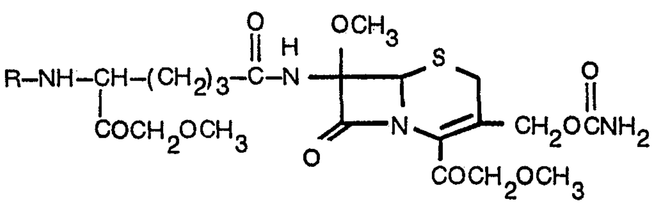

où R est un p-tosyle dans un solvant inerte comme le chlorure de méthylène à une température

comprise entre 50 et 90°C, le perfectionnement impliquant de conduire la réaction en présence de (1) environ 0,8 à 1,5 équivalent de triméthylsilyl-alcoyle inférieur-carbamate ou (2) d'environ 0,8 à 4 équivalents de triméthylsilyl-énol-éther

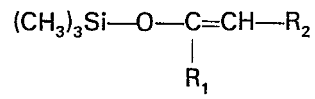

II

où $R_1$ peut être un alcoyle inférieur en $C_1$ à $C_4$; $R_2$ est un hydrogène ou un alcoyle inférieur en $C_1$ à $C_4$; ou $R_1$ et $R_2$ peuvent être une chaîne alcoylène réunie ayant de 2 à 4 atoms de carbone.

2. Procédé de la revendication 1 où le triméthylsilyl-alcoyle inférieur-carbamate à de 1 à 4 atomes de carbone dans l'alcoyle inférieur.

3. Procédé de la revendication 1 où l'on utilise le triméthylsilyl-méthyl-carbamate.

4. Procédé de la revendication 1 où l'on utilise le triméthylsilyl-éthyl-carbamate.

5. Procédé de la revendication 1 où le triméthylsilyl-énol-éther est le 2-triméthylsiloxyl-propène.

6. Procédé de la revendication 1 où le triméthylsilyl-énol-éther est le 1-triméthylsiloxyl-cyclohexène.

**Patentansprüche**

1. In dem Verfahren zur Herstellung der Verbindung

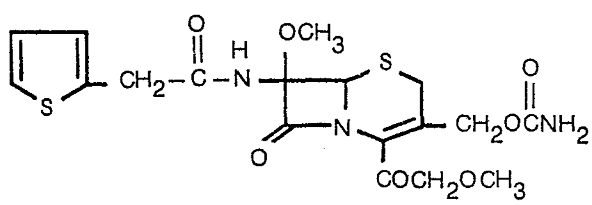

durch Umsetzung von Thienylacetylchlorid mit

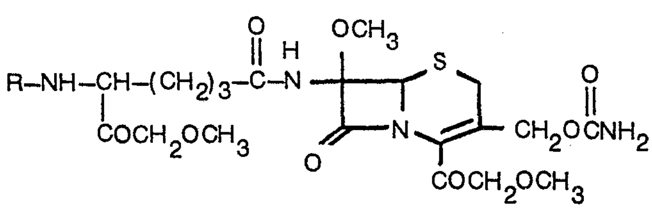

worin R p-Tosyl ist, in einem inerten Lösungsmittel, wie Methylenchlorid, bei einer Temperatur zwischen 50 und 90°C, die Verbesserung, welche die Ausführung der Umsetzung in Gegenwart von entweder (1) etwa 0,8 bis 1,5 Äquivalenten Trimethylsilyl-niedrigalkylcarbamat oder (2) etwa 0,8 bis 4 Äquivalenten Trimethylsilylenolether

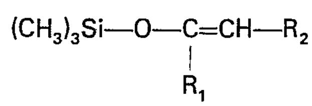

II

umfaßt, worin $R_1$ Niedrigalkyl mit 1—4 Kohlenstoffatomen sein kann; $R_2$ Wasserstoff oder Niedrigalkyl mit 1—4 Kohlenstoffatomen ist; oder $R_1$ und $R_2$ gemeinsam eine Alkylenkette mit 2—4 Kohlenstoffatomen sein können.

2. Das Verfahren nach Anspruch 1, in welchem das Trimethylsilyl-niedrigalkylcarbamat 1—4 Kohlenstoffatome in dem Niedrigalkyl aufweist.

3. Das Verfahren nach Anspruch 1, in welchem Trimethylsilylmethylcarbamat verwendet wird.

4. Das Verfahren nach Anspruch 1, in welchem Trimethylsilylethylcarbamat verwendet wird.

5. Das Verfahren nach Anspruch 1, in welchem der Trimethylsilylenolether 2-Trimethylsiloxylpropen ist.

6. Das Verfahren nach Anspruch 1, in welchem der Trimethylsilylenolether 1-Trimethylsiloxylcyclohexen ist.